# EUROPEAN PATENT SPECIFICATION

(11) **EP 4 391 929 B1**
(45) Date of publication and mention of the grant of the patent: **04.11.2026**
(21) Application number: 22769855.2
(22) Date of filing: 25.08.2022
(51) Int. Cl.: A61B 17/11, A61B 17/00

(54) **ANCHORS FOR IMPLANTABLE MEDICAL DEVICES**
ANKER FÜR IMPLANTIERBARE MEDIZINISCHE VORRICHTUNGEN
DISPOSITIFS D'ANCRAGE POUR DISPOSITIFS MÉDICAUX IMPLANTABLES

(30) Priority: 27.08.2021 US 202163237902 P
(43) Date of publication of application: 03.07.2024
(73) Proprietor: W. L. Gore & Associates, Inc., Newark, DE 19711 (US)
(72) Inventor: GILL, Kevin A., Newark, Delaware 19711 (US); LIN, Min-Hsuan, Newark, Delaware 19711 (US); MARYN, Rebecca L., Newark, Delaware 19711 (US); MINOR, David J., Newark, Delaware 19711 (US); SHAW, Edward E., Newark, Delaware 19711 (US); TRAPP, Benjamin M., Newark, Delaware 19711 (US)
(74) Representative: HGF
(86) International application number: PCT/US2022/041455
(87) International publication number: WO 2023/028189

(56) References cited:
- WO-A1-01/17435
- WO-A1-2017/074652
- WO-A1-2020/018699
- US-A1- 2016 045 199
- US-A1- 2019 328 374

## Description

### CROSS-REFERENCE TO RELATED APPLICATION

This application claims the benefit of Provisional Application No. 63/237,902, filed August 27, 2021.

### BACKGROUND

Implantable medical devices are often anchored in the body of a patient using an associated anchor. The anchor may be integral with other portions of the medical device, or may be a separate component that is attached to the medical device. Medical devices with which anchors are associated include grafts, stent grafts, pumps, or any of a variety of other medical devices that are to be held in place in the body. There are a variety of bodily structures to which medical devices may be anchored, such as the vasculature, organs, or other bodily structures. And, such medical devices may be configured to meet any of a variety of treatment objectives. For example, anchored medical devices may be utilized as occluders to treat atrial septal defects, patent forum ovale, or other conditions in intestines, fallopian tubes, or other bodily structures. Additional examples of anchored medical devices include those that help facilitate movement of fluids within a patient, such as shunts, prosthetic valves, and others. Still other types of implantable devices may include devices for monitoring and/or controlling physiologic conditions, such as smart medical devices.

WO2017/074652 A1 describes an implantable medical device for sealing and repairing defects in a body tissue or for creating an anastomosis includes a frame and a covering material. The frame includes a single continuously wound wire that defines an apposition portion, a defect-occupying portion, and a sealing portion

US2019/328374 A1 describes am implantable closure device comprising a self-expanding metal frame comprising a central portion defining a lumen and a plurality of first anchoring arms and a plurality of second anchoring arms angularly spaced around the central portion.

WO01/17435 A1 describes a septal defect closure device having a first occluding disk having a first flexible membrane attached to a first frame and a second occluding disk having a second flexible membrane attached to a separate second frame.

WO2020/018699 A1 describes an implantable medical device comprising a first frame component. The first frame component including a first set of elongate elements configured to conform to an anatomy of a patient. The implantable medical device also comprising a second frame component including a second set of elongate elements configured to conform to an anatomy of a patient. The first frame component and the second frame component being discrete and separate from one another.

### SUMMARY

The invention is defined according to the appended claims in which there is described:

An anchor for an implantable medical device, the anchor comprising: one or more wires arranged to form: a first frame component including a plurality of first contact features defining a plurality of first outer apices and a plurality of first inner apices arranged between each of the plurality of first contact features; and a second frame component including a plurality of second contact features defining a plurality of second outer apices and a plurality of second inner apices arranged between each of the plurality of second contact features, the plurality of first contact features and the plurality of second contact features are positioned opposite one another such that the plurality of first contact features and the plurality of second contact features are angularly aligned with one another about a central longitudinal axis of the anchor; the one or more wires including a substantially linear section extending between each of the plurality of first outer apices and a substantially linear section extending between each of the plurality of second outer apices such that the plurality of first contact features each have approximately a diamond shape and the plurality of second contact features each have approximately a diamond shape; and an intermediate section including a membrane coupling the first frame component and the second frame component, the first frame component arranged on a first side of the intermediate section and the second frame component arranged on a second side of the intermediate section, the inner apices of each of the plurality of first and second contact features being adjacent to and spaced about a circumference of the intermediate section, wherein the one or more wires includes a continuous wind pattern forming the first frame component, the second frame component, and the intermediate section.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 is an example anchor that may be associated with an implantable medical device in accordance with an embodiment.
FIG. 2 is an example frame component that may form a part of an anchor that may be associated with an implantable medical device in accordance with an embodiment.
FIG. 3 is an example frame component that may form a part of an anchor that may be associated with an implantable medical device and a winding configuration in accordance with an embodiment.
FIG. 4 is an example anchor associated with an implantable medical device in accordance with an embodiment.
FIG. 5 is an example medical device that may be associated with an anchor in accordance with an embodiment.
FIG. 6 is another example anchor that may be associated with an implantable medical device in accordance with an embodiment.
FIG. 7 is another example anchor that may be associated with an implantable medical device in accordance with an embodiment.
FIG. 8 is another example anchor that may be associated with an implantable medical device in accordance with an embodiment.
FIG. 9 is another example anchor that may be associated with an implantable medical device in accordance with an embodiment.
FIG. 10 is an example anchor in accordance with an embodiment.
FIG. 11 is an example anchor and an implantable medical device in accordance with an embodiment.
FIG. 12 is an example anchor and an implantable medical device in accordance with an embodiment.

### DETAILED DESCRIPTION

### Definitions and Terminology

This disclosure is not meant to be read in a restrictive manner. For example, the terminology used in the application should be read broadly in the context of the meaning those in the field would attribute such terminology.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. In the event it is determined that individuals having ordinary skill in the relevant arts would not readily ascertain values for such reasonably small differences, the terms "about" and "approximately" can be understood to mean plus or minus 10% of the stated value.

### Description of Various Embodiments

Persons skilled in the art will readily appreciate that various aspects of the present disclosure can be realized by any number of methods and apparatuses configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

Various aspects of the present disclosure are directed toward anchoring an implantable medical device within a patient and medical devices for use within a patient. In certain instances, the embodiments of implantable medical device herein may be associated with (e.g., attached to and/or integrally formed with) an implantable medical device. The implantable medical device, which may be an occluder, may additionally be arranged with a shunt to treat heart failure when placed across an atrial septum, or any of a variety of other locations. These are examples only, and the anchors and various designs associated therewith may be used with such implantable medical devices implanted in a variety of locations in the body, such as shunts, prosthetic valves, and devices for monitoring and/or controlling physiologic conditions, such as smart medical devices, for example. The anchors, as discussed herein, may facilitate anchoring the medical device and minimize risk of embolization, minimize risk of creating loose thrombus, and/or minimize risk of enabling loose tissue growth - all of which can cause stroke. The apparatuses, systems, and methods are directed toward a catheter-delivered anchors for implantable medical devices or medical devices that anchor to structures in a patient.

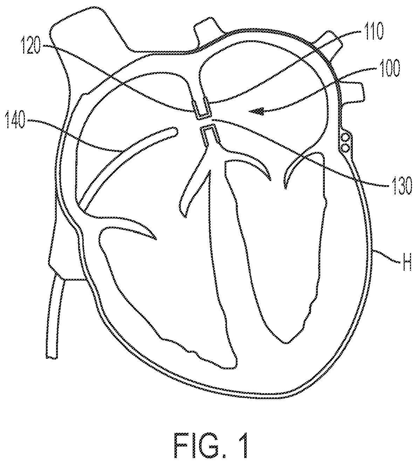

FIG. 1 is an anchoring implantable medical device in accordance with an embodiment. The anchor 100 is shown implanted within a heart H of a patient. The device 100 is shown arranged between the patient's left atrium and right atrium. In certain instances, the device 100 may be used to regulate blood pressure or pressures within the heart H, for example, between the left and right atria LA, RA. As shown, the device 100 generally includes a first frame component 110 arranged on a first side of a septum (e.g., within the right atrium RA), a second frame component 120 arranged on a second side of the septum (e.g., within the left atrium LA), and an intermediate section 130 extending through the septum. A valve may be placed within intermediate section 130 to assist in the regulation of blood pressures within the heart H. A needle may be used to create an opening in the septum. The designs may be used to anchor any of a variety of implantable medical devices at the location shown, including occluders, fenestrated occluders, and others. And, though the example of anchoring in a heart, and specifically a septal wall is depicted, as previously mentioned, any of a variety of locations within the body and associated treatments may benefit from the anchor designs disclosed herein.

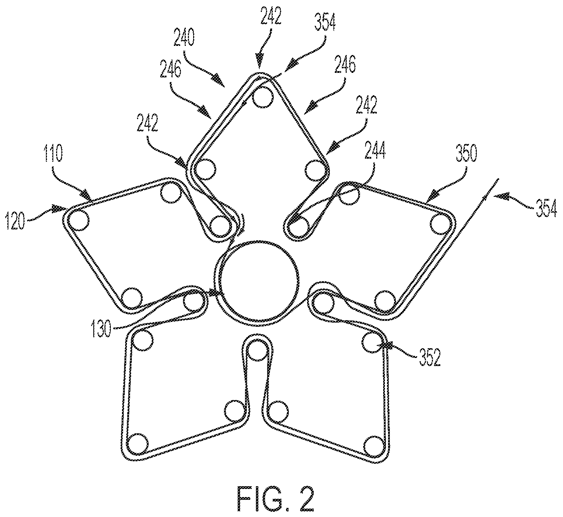

A sheath 140 and constraining and/or release lines (not shown) may be used to facilitate deployment of the device 100. For example, a first side of the device 100 that includes the first frame component 110 may be released after the sheath 140 is advanced through the septum and to the LA, and the second frame component 120 that includes the second frame component 120 may be released on the RA side of the septum. An intermediate section 130 (e.g., shown in FIG. 2) is arranged within the opening. The frame components 110, 120 and/or the intermediate section 130 may be compressed within the sheath 140 during delivery of the device 100 to the desired treatment area within the patient and subsequently expanded during deployment of the device 100. In other instances, the anchor 100 may be implanted at a different location in the heart or a different location within the patient (e.g., gastrointestinal system, vasculature, brain).

In certain instances, the intermediate section 130 is configured to be associated with at least a portion of an implantable medical device 500. The intermediate section 130 may be associated with (e.g., integrally formed with, attached, or otherwise associated with) the medical device 500 according to various examples. The intermediate section 130, in certain instances, may extend beyond each of the first frame component 110 and the second frame component 120 and between the first frame component 110 and the second first frame component 120 to facilitate anchoring of the implantable medical device 500 or to manage flow of blood, thrombus formation, or the like.

FIG. 2 shows an example winding pattern of first frame component 110 and second frame component 120. In particular, FIG. 2 shows two passes of a wire about a winding pattern to define the first frame component 110 and the second frame component 120, as well as engagement section 130 between the first and second frame components. The first frame component 110 is shown as the inner winding and the second frame component 120 is slightly offset and shown as an outer winding. This is done for illustrative purposes, as this is a two-dimensional representation of a three-dimensional object, and the windings may have essentially the same size according to various embodiments. And, although both the first and second frame components 110, 120 are shown, only one frame component may take the general shape shown in FIG. 2, and the other may be absent, or take a different shape according to various examples. As shown, a single wire 350 may be wound about a plurality of winding pegs 352 to form the first frame component 110 (and second frame component 120) as desired.

First frame component 110 (and second frame component 120) may form a part of an anchor for anchoring of an implantable medical device 500 in accordance with an embodiment. The shape shown for the first frame component 110 and/or the second frame component 120 may be the shape and configuration of the first frame component 110 and/or the second frame component 120 shown in FIGS. 1, 10, and 11. As shown, the first frame component 110 may include one or more wires, as can the second frame component 120. As described in further detail below, the one or more wires may form the first frame component 110, the second frame component 120, and an intermediate section 130 between the first and second frame components (e.g., as shown in FIGS. 1, 10, and 11) of an anchor. The first frame component 110 may be arranged on a first side of the intermediate section 130 and the second frame component 120 may be arranged on a second side of the intermediate section 130 so that the intermediate section 130 is between or substantially between the frame components.

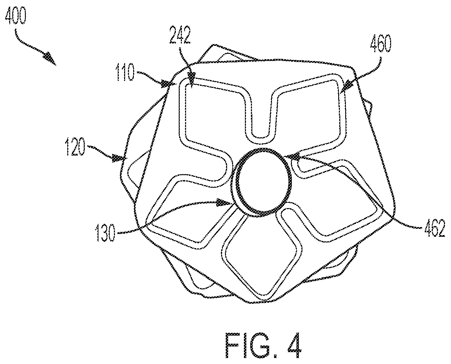

Each of the first frame component 110 and the second frame component 120 may include a plurality of contact features 240, one location for which is highlighted in FIG. 2 for ease of illustration. Contact features are suitable for contact with structures within the patient. Embodiments with contact features may be configured to allow tissue ingrowth to enhance long term anchoring and to be atraumatic to the tissue they contact to reduce incidences of tissue erosion, which in some embodiments may be accomplished by covering contact features with films. For example, a membrane 460, also described as a covering, may be arranged across the contact features 242 of the first frame component 110 and the contact features 242 of the second frame component 120 as shown and subsequently described in association with FIG. 4.

In instances where an anchor includes the first frame component 110 and the second frame component 120, the contact features 240 of the first frame component 110 may be a first plurality of contact features 240 and the contact features 240 of the second frame component 120 may be a second plurality of contact features 240. The contact features 240, in certain instances, may include a plurality of outer apices 242 and at least one inner apex 244 arranged between each of the plurality of contact features 240. In instances where an anchoring device includes the first frame component 110 and the second frame component 120, the plurality of outer apices 242 and the at least one inner apex 244 of the first frame component 110 may be a first plurality of outer apices 242 and at least one first inner apex 244 and the plurality of outer apices 242 at least one inner apex 244 of the second frame component 120 may be a plurality of second outer apices 242 and at least one second inner apex 244.

In certain instances, the one or more wires that form the frame component 100 include a substantially linear section 246 between each of the plurality of outer apices 242. The substantially linear section 246 between each of the plurality of outer apices 242 may form approximately a diamond shape of the contact features 242 as is shown in FIG. 2. In addition, the plurality of contact features 242 may be an odd number of contact features 242 (e.g., three, five, seven) in certain instances or an even number of contact features 242 (e.g., two, four, six, eight) in other instances. In certain instances, the at least one inner apex 244 of each of the plurality of contact features 242 is adjacent to and spaced about a circumference of the intermediate section 130. In certain instances, the outer apices 242 and at least one inner apex 244 have curvatures.

In certain instances, the plurality of outer apices 242 may be used to engage deployment tethers or portions of a deployment system. The plurality of outer apices 242 may also engage retrieval loops to allow for recapture or retrieval of the device after it is deployed. The deployment system may use one or more constraining lines and/or release lines as discussed in further detail in WO2020018697A1 (Cole et al.).

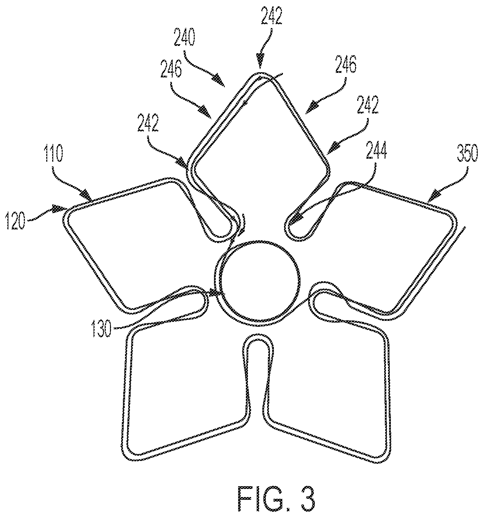

FIG. 3 shows the first frame component 110 and the second frame component 120 that may form a part of an anchor configured to anchor an implantable medical device and a winding configuration in accordance with an embodiment. In FIG. 3, the winding pegs 352 are not shown, and the ends of the wire 350 have been trimmed to the final frame shape. Though one wire is shown in FIGS. 2 and 3, the first frame component 110 may be formed of one or more wires 350.

As shown in FIG. 2, in certain instances, the wire(s) 350 may including a starting point 354 and an ending point 354 in forming a first frame component 110. In certain instances, the starting point 354 of the wire(s) 350 may be within the first frame component 110 and the ending point 354 may be within a second frame component 120. In addition, the wire(s) 350 may form the intermediate section 130. In certain instances, the intermediate section 130 includes a circumference that includes at least one turn of the wire(s) 350. The wire(s) 350 may include at least one wire 350 forming the first plurality of contact features 242, the section 130, and the second plurality of contact features 242. The wire(s) 350 includes a continuous wind pattern in forming the first frame component 110, the second frame component 120, and the intermediate section 130.

As shown, the first frame component 110 includes five contact features 242 each of which include a diamond shape. In certain instances, the five contact features 242 (or an odd number of contact features) may resist prolapse when implanted in the atrial septum (or target area of the body/patient). The shape and arrangement of the contact features 242 may resist prolapse and stabilize an implantable medical device 500 arranged within the intermediate section 130. The odd number of contact features 242 and shape of the contact features may facilitate crush or constraining of the contact features 242 while also minimizing stress, minimizing prolapse, and allowing for retrievability. In certain instances, the frame components 110, 120 may be substantially planar or may be concave or convex.

FIG. 4 is an example anchor 400 for an implantable medical device in accordance with an embodiment. The anchor 400 may be part of a system that includes an implantable medical device 500 (sensor, pump, lead, or other medical device). As discussed in detail above, the anchor 400 includes an intermediate section 130 configured to allow placement of an implantable medical device 500 within the section 130, a first frame component 110 arranged on a first side of the intermediate section 130, which may include an odd number of contact features 242 each having a portion with an approximate diamond shape, and a second frame component 120 arranged on a second side of the intermediate section 130, which may include an odd number of contact features 242 each having a portion with an approximate diamond shape.

In certain instances, the first frame component 110, the second frame component 120, and the intermediate section 130 are formed by at least one wire. As shown in FIGS. 2 and 3, the wire(s) 350 includes a continuous wind pattern in forming the first frame component 110, the second frame component 120, and the intermediate section 130. In certain instances, the first frame component 110 and the second frame component 120 are formed from separate wires with separate membranes. The first frame component 110 and the second frame component 120 may be separate from one another. In other instances, the intermediate section 130 may be formed of a membrane that separates the first frame component 110 and the second frame component 120. In addition, one or both of the first frame component 110 and the second frame component 120 may be concave.

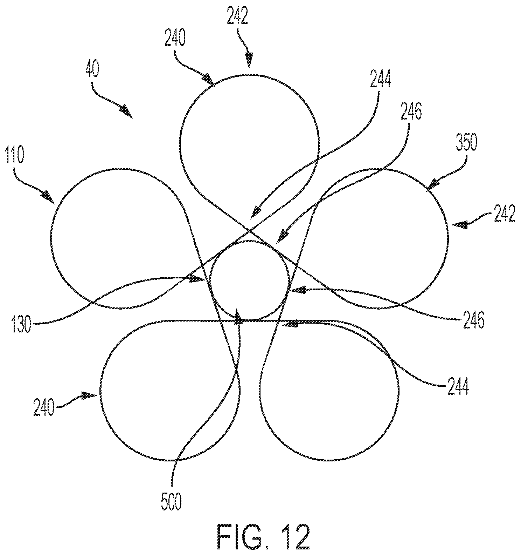

In certain instances, and as is shown in FIG. 4, the contact features 242 of the first frame component 110 and the contact features 242 of the second frame component 120 are aligned (e.g., relative to a longitudinal axis of the intermediate section 130). The contact features 242 of each of the frame components 110, 120 being allowed may allow for crossing or re-crossing of the anchor 400 after implantation. In certain instances, the anchor 400 includes a membrane 460 coupled to the first frame component 110 and the second frame component 120. The membrane 460 may be arranged across the contact features 242 of the first frame component 110 and the contact features 242 of the second frame component 120. As shown in FIG. 4, the membrane 460 spans areas 462 of the first frame component 110 and the second frame component 120 that do not include the at least one wire that forms the first frame component 110 and the second frame component 120. As noted above, the contact features 242 of the first frame component 110 and the contact features 242 of the second frame component 120 are aligned. The areas 462 of the membrane where there are no wire(s) may be punctured for recrossing (e.g., for a future procedure such as ablation, left atrial appendage device implantation). Similar concepts apply to the designs shown in FIGS 2 and 12, where the membrane 460 may be included and cover the contact features 242 of those designs. And, similarly there, the membrane where there are no wire(s) may be punctured for re-crossing (e.g., for a future procedure such as ablation, left atrial appendage device implantation)

In implanting the anchor 400, which may be used in a method of managing treatment of a patient with congestive heart failure, occluding a defect in the wall of a body structure, or for other treatment as desired. As shown, the anchor 400 may be placed on a tissue (e.g., atrial septum) of the patient. The first frame component 110 may be arranged on a first side of the septum and the second frame component 120 may be arranged on a second side of the septum. After deployment of the anchor 400, an implantable medical device 500 may be arranged within intermediate section 130 or the opening defined by intermediate section 130 in the embodiments may be increased to allow blood flow to treat heart failure or the opening defined by intermediate section 130 may be decreased such that blood flow would not occur and a defect is occluded. In other instances, the implantable medical device 500 may be built with the anchor 400 and delivered in place.

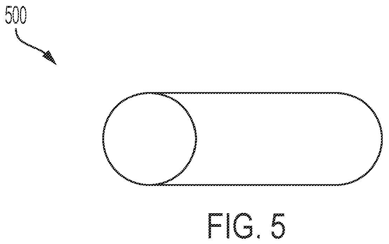

Fig. 5 includes a generalized view of an implantable medical device 500 that may be coupled to the anchor 400 and anchored within the body of a patient. The implantable medical device 500 may be any of a variety of devices, such as a stent graft, stent, valve, pump, lead, or other type of medical device including smart medical devices with health monitoring capabilities or the like.

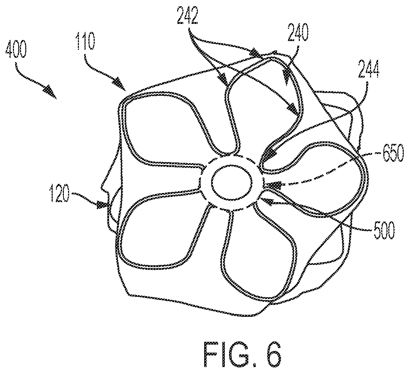

FIG. 6 is another example anchor 400 for anchoring an implantable medical device in accordance with an embodiment or for treatment of a condition in a patient such as occluding a defect. The anchor 400 may anchor a pump, lead, or other type of medical device that can be anchored within a patient. The first frame component 110 and second frame component 120 are shown in FIG. 6, where the features of the second frame component 120 are partially hidden by the first frame component 110. It should also be clear that the second frame component 120 may have similar features to those shown for the first frame component 110.. The first frame component 110 may include one or more wires. The one or more wires may form the first frame component 110, the second frame component 120, and an intermediate portion (e.g., intermediate section 130 shown in FIG. 1). In other instances, the intermediate section 130 may include a graft material arranged between and connecting the first frame component 110 and the second frame component 120.The first frame component 110 may be arranged on a first side of the intermediate portion and the second frame component 120 may be arranged on a second side of the intermediate portion.

The first frame component 110 may include a plurality of contact features 240, one of which is highlighted in FIG. 6 for ease of illustration. In instances where an implantable device includes the first frame component 110 and the second frame component 120, the contact features 240 of the first frame component 110 may be a first plurality of contact features 240 and the contact features 240 of the second frame component 120 may be a second plurality of contact features 240. The contact features 240, in certain instances, may include a plurality of outer apices 242 and a plurality of inner apices 244 arranged between each of the plurality of contact features 240. In instances where an implantable device includes the first frame component 110 and the second frame component 120, the plurality of outer apices 242 and the plurality of inner apices 244 of the first frame component 110 may be a first plurality of outer apices 242 and the plurality of inner apices 244 and the plurality of outer apices 242 and the plurality of inner apices 244 of the second frame component 120 may be a plurality of second outer apices 242 and a plurality of second inner apices 244.

The inner apices 244 may define inner engagement features for engagement with one or more features of a medical device. In certain instances, and as is shown, the plurality of first inner apices 244 and/or the plurality of second inner apices 244 may be configured to anchor an implantable medical device 500. The anchor 400 may be associated with the implantable device in a variety of ways, including via the intermediate section 130, also described as an intermediate portion or central portion of the anchor. The shape of the intermediate section 130 may take on a variety of shapes for a variety of purposes, including anchor performance, ease of manufacture, and other functions. For example, the shape of the intermediate section 130 may promote better flexing and/or fatigue reliability, may facilitate association with medical devices of differing configurations, or others.

In certain instances, the portions of the one or more wires that form the first frame component 110 are uncoupled from portions of the one or more wires that form the second frame component 120 are unconnected.

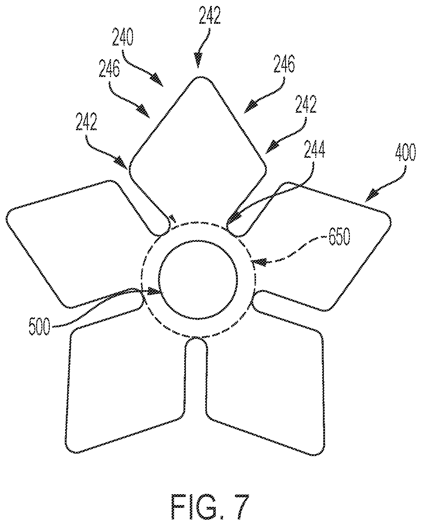

FIG. 7 is another example anchor 400. The anchor 400 may anchor a pump, lead, or other type of medical device that can be anchored within a patient. The first frame component 110 shown in FIG. 6 may be the first frame component 110 and/or the second frame component 120 (e.g., as shown in FIGS. 1, 10, and 11). The first frame component 110 may include one or more wires. The one or more wires may form the first frame component 110, the second frame component 120, and an intermediate portion (e.g., engagement section shown in FIG. 1).A membrane may cover the first frame and second frame components. In other instances, the intermediate portion may include a graft material arranged between and connecting the first frame component 110 and the second frame component 120.The first frame component 110 may be arranged on a first side of the intermediate portion and the second frame component 120 may be arranged on a second side of the intermediate portion.

The first frame component 110 may include a plurality of contact features 240, one of which is highlighted in FIG. 7 for ease of illustration. In instances where an implantable device includes the first frame component 110 and the second frame component 120, the contact features 240 of the first frame component 110 may be a first plurality of contact features 240 and the contact features 240 of the second frame component 120 may be a second plurality of contact features 240. The contact features 240, in certain instances, may include a plurality of outer apices 242 and a plurality of inner apices 244 arranged between each of the plurality of contact features 240. In instances where an implantable device includes the first frame component 110 and the second frame component 120, the plurality of outer apices 242 and the plurality of inner apices 244 of the first frame component 110 may be a first plurality of outer apices 242 and the plurality of inner apices 244 and the plurality of outer apices 242 and the plurality of inner apices 244 of the second frame component 120 may be a plurality of second outer apices 242 and a plurality of second inner apices 244.

In certain instances, the portions of the one or more wires that form the first frame component 110 are uncoupled from portions of the one or more wires that form the second frame component 120 and are unconnected. In addition, the first frame component 110 and the second frame component 120 may be separated by an intermediate portion (e.g., as shown in FIGS. 1, 10, and 11). The intermediate portion may be formed by portions of the wires and/or by a graft material arranged between and connecting the first frame component 110 and the second frame component 120.

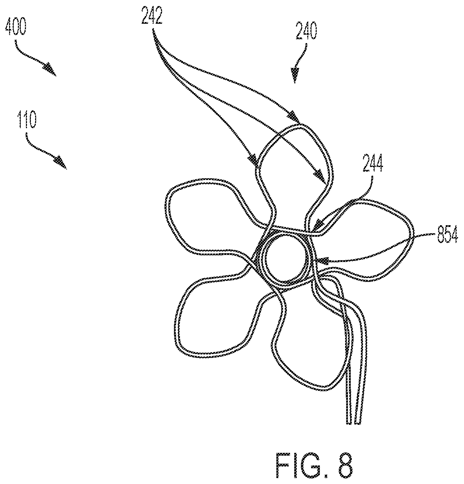

FIG. 8 is another example implantable medical device including an anchor 400 in accordance with an embodiment. The anchor 400 may anchor a pump, lead, or other type of medical device that can be anchored within a patient. The first frame component 110 shown in FIG. 8 may be the first frame component 110 and/or the second frame component 120 (e.g., as shown in FIGS. 1, 10, and 11). The first frame component 110 may include one or more wires. The one or more wires may form the first frame component 110, the second frame component 120, and an intermediate portion (e.g., engagement section shown in FIG. 1). In other instances, the intermediate portion may include a graft material arranged between and connecting the first frame component 110 and the second frame component 120.The first frame component 110 may be arranged on a first side of the intermediate portion and the second frame component 120 may be arranged on a second side of the intermediate portion.

The first frame component 110 may include a plurality of contact features 240, one of which is highlighted in FIG. 8 for ease of illustration. In instances where an implantable device includes the first frame component 110 and the second frame component 120, the contact features 240 of the first frame component 110 may be a first plurality of contact features 240 and the contact features 240 of the second frame component 120 may be a second plurality of contact features 240. The contact features 240, in certain instances, may include a plurality of outer apices 242 and a plurality of inner apices 244 arranged between each of the plurality of contact features 240. In instances where an implantable device includes the first frame component 110 and the second frame component 120, the plurality of outer apices 242 and the plurality of inner apices 244 of the first frame component 110 may be a first plurality of outer apices 242 and the plurality of inner apices 244 and the plurality of outer apices 242 and the plurality of inner apices 244 of the second frame component 120 may be a plurality of second outer apices 242 and a plurality of second inner apices 244.

In certain instances, the portions of the one or more wires that form the first frame component 110 are uncoupled from portions of the one or more wires that form the second frame component 120 and are unconnected. For example, the one or more wires may form a first section 854 forming a boundary extending between the plurality of first inner apices 244 configured to connect to the implantable medical device 500 within the first frame component 110. The second frame component 120 may also include a second section 854 formed by portions of the one or more wire that may also be configured to connect to the implantable medical device 500 within the second frame component 120. In certain instances, the first inner engagement section 854 and/or the second inner engagement section 854 may have approximately a circular shape or other shape to conform to the outer shape of the medical device to be anchored. The first section 854 and/or the second section 854 may be configured to limit rotational freedom of the implantable medical device 500 when anchored.

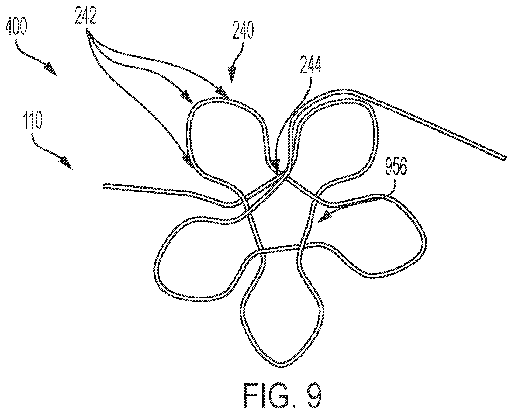

FIG. 9 is another example implantable anchor for a medical device in accordance with an embodiment. The anchor 400 may anchor a pump, lead, or other type of medical device that can be anchored within a patient. The first frame component 110 shown in FIG. 9 may be the first frame component 110 and/or the second frame component 120 (e.g., as shown in FIGS. 1, 10, and 11). The first frame component 110 may include one or more wires. The one or more wires may form the first frame component 110, the second frame component 120, and an intermediate portion (e.g., intermediate section 130 shown in FIG. 1). In other instances, the intermediate portion may include a graft material arranged between and connecting the first frame component 110 and the second frame component 120.The first frame component 110 may be arranged on a first side of the intermediate portion and the second frame component 120 may be arranged on a second side of the intermediate portion.

The first frame component 110 may include a plurality of contact features 240, one of which is highlighted in FIG. 9 for ease of illustration. In instances where an implantable device includes the first frame component 110 and the second frame component 120, the contact features 240 of the first frame component 110 may be a first plurality of contact features 240 and the contact features 240 of the second frame component 120 may be a second plurality of contact features 240. The contact features 240, in certain instances, may include a plurality of outer apices 242 and a plurality of inner apices 244 arranged between each of the plurality of contact features 240. In instances where an implantable device includes the first frame component 110 and the second frame component 120, the plurality of outer apices 242 and the plurality of inner apices 244 of the first frame component 110 may be a first plurality of outer apices 242 and the plurality of inner apices 244 and the plurality of outer apices 242 and the plurality of inner apices 244 of the second frame component 120 may be a plurality of second outer apices 242 and a plurality of second inner apices 244.

In certain instances, the portions of the one or more wires that form the first frame component 110 are uncoupled from portions of the one or more wires that form the second frame component 120 are unconnected. In addition, the one or more wires may also form a first section 854 forming a boundary extending between the plurality of first inner apices 244 . The second frame component 120 may also include a second section 854 formed by portions of the one or more wires. The first section 854 and/or the second section 854 may be configured to be associated with an implantable medical device. In certain instances, the first section 854 and/or the second section 854 may have approximately a polygonal shape.

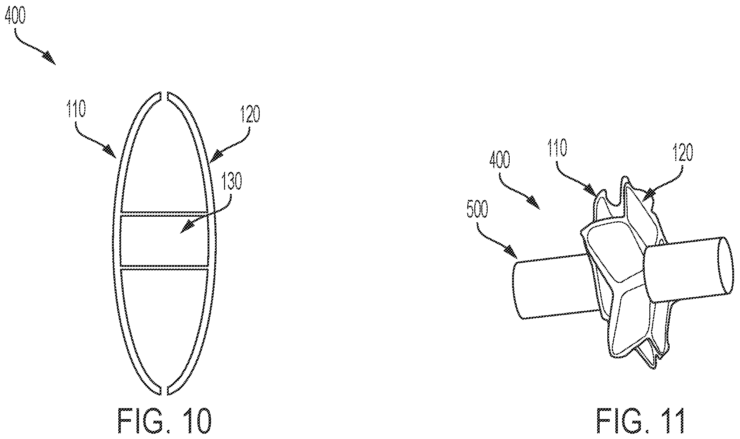

FIGS. 10 and 11 show a configuration in which the first frame component 110 and the second frame component 120 are curved (e.g., convexly curved), or are otherwise curved toward one another when the anchor is in an unbiased, or unconstrained state. FIG. 10 is a schematic representation of this feature, and FIG. 11 shows one example of an anchor 400 so configured. In certain instances, the first frame component 110 and the second frame component 120 are curved inwardly relative to the intermediate section 130. The curved frame components 110, 120 may facilitate enhanced contact with a target tissue location, lessening of the opportunity for obstructive tissue growth along the curved frame components 110, 120 that could interfere with implantable medical device 500 performance, and/or reduce or avoid other detrimental effects, such as thrombus formations, and others. In some examples, the curvature of the anchor frame components 110, 120 enhances apposition against an interposed bodily structure (e.g., tissue), providing better potential contact of outer apices 242 without unwanted spacing between the contact features 240 and the interposed structure (e.g., without "coning" or "tenting" of the anchor 400. In some examples, the curved frame components 110, 120 are configured such that the plurality of first outer apices 242 and the plurality of second outer apices 242 are angled toward one another when the anchor 400 is in an unconstrained state.

FIG. 12 shows still another configuration of anchor 400 according to various embodiments. As shown, the anchor 400 includes at least one wire 350 defining a plurality of contact features 240 in the form closed wire loops, where the wire loops close by overlapping at the inner apices 244. Only the first frame component 110 is visible in FIG. 12, but the second frame component 120 may have substantially the same configuration, or may differ either in size or shape, or in orientation (e.g., by being rotationally offset from the first frame component 110). The contact features 240 define a plurality of outer apices 242. Each of the inner apices 244 of the contact features 240 are connected to two adjacent ones of the inner apices 244. As shown, they are connected by a linear section 246 extending between each of the plurality of outer apices 242. This plurality of linear sections 246 may define the intermediate section 130, as shown, which is associated with implantable medical device 500. Also, as shown, the closed wire loops defined by the contact features 240 are positioned outside, and do not overlap with one another outside of the inner apices 244, nor do they overlap inside of the intermediate section 130. In other words, the plurality of contact features 240 define a plurality of non-overlapping closed loops disposed about the central longitudinal axis of the anchor 400, each of the non-overlapping closed loops being connected to two adjacent ones of the closed loops by a plurality of linear sections 246.

With the foregoing in mind, an anchor 400 according to the design of FIG. 12 may include first frame component 110 including plurality of first contact features 240 including plurality of first outer apices 242 and plurality of first inner apices 244 arranged between each of the plurality of first contact features 240. The first frame component 110 may include one or more wound wires 350 forming the plurality of first contact features 240 and the plurality of first contact features may include a plurality of linear sections 246 between each of the plurality of first inner apices 244. The plurality of first contact features 240 may forming a plurality of first closed loops disposed about the central longitudinal axis of the anchor, each of the non-overlapping first closed loops being connected to two adjacent ones of the first closed loops by a plurality of first linear sections 246.

Second frame component 120 may be similarly configured, with the one or more wound wires 350 forming plurality of second contact features 240 including second linear sections 356 between each of the second inner apices 244, the plurality of second contact features 240 forming a plurality of second closed loops disposed about the central longitudinal axis of the anchor, each of the non-overlapping second closed loops being connected to two adjacent ones of the second closed loops by a plurality of second linear sections 246.

And, as previously referenced, the plurality of first linear sections 246 (and/or the plurality of second linear sections 246) may define intermediate region 130 between the first frame component 110 and the second frame component 120.

A membrane material, discussed herein, may include a fluoropolymer, such as a polytetrafluoroethylene (PTFE) polymer or an expanded polytetrafluoroethylene (ePTFE) polymer. In some instances, the membrane material may be formed of, such as, but not limited to, a polyester, a silicone, a urethane, a polyethylene terephthalate, or another biocompatible polymer, or combinations thereof. In some instances, bioresorbable or bioabsorbable materials may be used, for example a bioresorbable or bioabsorbable polymer. In some instances, the membrane material can include Dacron, polyolefins, carboxy methylcellulose fabrics, polyurethanes, or other woven, non-woven, or film elastomers.

In addition, nitinol (NiTi) may be used as the material of the frame (and any of the frames discussed herein), but other materials such as, but not limited to, stainless steel, L605 steel, polymers, MP35N steel, polymeric materials, Pyhnox, Elgiloy, or any other appropriate biocompatible material, and combinations thereof, can be used as the material of the frame. The super-elastic properties and softness of NiTi may enhance the conformability of the frame elements. In addition, NiTi can be shape-set into a desired shape. That is, NiTi can be shape-set so that the frame tends to self-expand into a desired shape when the frame is unconstrained, such as when the frame is deployed out from a delivery system.

## Claims

1. An anchor (100) for an implantable medical device, the anchor (100) comprising:
one or more wires (350) arranged to form:
a first frame component (110) including a plurality of first contact features (240) defining a plurality of first outer apices (242)and a plurality of first inner apices (244) arranged between each of the plurality of first contact features (240); and
a second frame component (120) including a plurality of second contact features (240) defining a plurality of second outer apices (242) and a plurality of second inner apices (244) arranged between each of the plurality of second contact features (240),
wherein the plurality of first contact features (240) and the plurality of second contact features (240) are positioned opposite one another such that the plurality of first contact features (240) and the plurality of second contact features (240) are angularly aligned with one another about a central longitudinal axis of the anchor;
the one or more wires including a substantially linear section (246) extending between each of the plurality of first outer apices (242) and a substantially linear section extending between each of the plurality of second outer apices (242) such that the plurality of first contact features (240) each have approximately a diamond shape and the plurality of second contact features (240) each have approximately a diamond shape; and
an intermediate section (130) including a membrane coupling the first frame component (110) and the second frame component (120), the first frame component (110) arranged on a first side of the intermediate section (130) and the second frame component (120) arranged on a second side of the intermediate section (130), the inner apices (244) of each of the plurality of first and second contact features (240) being adjacent to and spaced about a circumference of the intermediate section (130), wherein the one or more wires (350) includes a continuous wind pattern forming the first frame component (110), the second frame component (120), and the intermediate section (130).

2. The anchor of claim 1, where in the plurality of first contact features (240) includes an odd number of contact features and the plurality of second contact features (240) includes an odd number of contact features.

3. The anchor of claim 2,wherein the plurality of first contact features (240) includes five contact features (240) and the plurality of second contact features (240) includes five contact features.

4. The anchor of any one of claims 1-3, wherein the first (110) and second frame components (120) are connected by at least one turn of the one or more wires (350).

5. The anchor of any one of claims 1-4, wherein the first frame component (110) and the second frame component (120) are curved inwardly toward one another such that the plurality of first outer apices (242) and the plurality of second outer apices (242) are angled toward one another when the anchor (100) is in an unconstrained state.

6. The anchor of any one of claims 1 to 5, further comprising a membrane (460) spanning areas of the first frame component (110) and the second frame component (120) defined by the one or more wires (350) that form the first frame component (110) and the second frame component (120).

## Patentansprüche

1. Anker (100) für eine implantierbare medizinische Vorrichtung, wobei der Anker (100) Folgendes umfasst:
einen oder mehrere Drähte (350), die so angeordnet sind, dass sie Folgendes ausbilden:
eine erste Rahmenkomponente (110), die eine Vielzahl von ersten Kontaktmerkmalen (240), die eine Vielzahl von ersten äußeren Scheiteln (242) und eine Vielzahl von ersten inneren Scheiteln (244) definieren, die zwischen jedem der Vielzahl von ersten Kontaktmerkmalen (240) angeordnet sind, enthält; und
eine zweite Rahmenkomponente (120), die eine Vielzahl von zweiten Kontaktmerkmalen (240), die eine Vielzahl von zweiten äußeren Scheiteln (242) und eine Vielzahl von zweiten inneren Scheiteln (244) definieren, die zwischen jedem der Vielzahl von zweiten Kontaktmerkmalen (240) angeordnet sind, enthält,
wobei die Vielzahl von ersten Kontaktmerkmalen (240) und die Vielzahl von zweiten Kontaktmerkmalen (240) einander gegenüberliegend positioniert sind, sodass die Vielzahl von ersten Kontaktmerkmalen (240) und die Vielzahl von zweiten Kontaktmerkmalen (240) winklig zueinander um eine zentrale Längsachse des Ankers ausgerichtet sind;
wobei der eine oder die mehreren Drähte einen im Wesentlichen linearen Abschnitt (246), der sich zwischen jedem aus der Vielzahl von ersten äußeren Scheiteln (242) erstreckt, und einen im Wesentlichen linearen Abschnitt, der sich zwischen jedem aus der Vielzahl von zweiten äußeren Scheiteln (242) erstreckt, enthalten, sodass die Vielzahl von ersten Kontaktmerkmalen (240) jeweils ungefähr eine Diamantform aufweisen und die Vielzahl von zweiten Kontaktmerkmalen (240) jeweils ungefähr eine Diamantform aufweisen; und
einen Zwischenabschnitt (130), der eine Membran enthält, die die erste Rahmenkomponente (110) und die zweite Rahmenkomponente (120) koppelt, wobei die erste Rahmenkomponente (110) auf einer ersten Seite des Zwischenabschnitts (130) angeordnet ist und die zweite Rahmenkomponente (120) auf einer zweiten Seite des Zwischenabschnitts (130) angeordnet ist, wobei die inneren Scheitel (244) jedes aus der Vielzahl von ersten und zweiten Kontaktmerkmalen (240) benachbart zu und beabstandet um einen Umfang des Zwischenabschnitts (130) sind, wobei der eine oder die mehreren Drähte (350) ein kontinuierliches Wicklungsmuster enthalten, das die erste Rahmenkomponente (110), die zweite Rahmenkomponente (120) und den Zwischenabschnitt (130) ausbildet.

2. Anker nach Anspruch 1, wobei die Vielzahl von ersten Kontaktmerkmalen (240) eine ungerade Anzahl von Kontaktmerkmalen enthält und die Vielzahl von zweiten Kontaktmerkmalen (240) eine ungerade Anzahl von Kontaktmerkmalen enthält.

3. Anker nach Anspruch 2, wobei die Vielzahl von ersten Kontaktmerkmalen (240) fünf Kontaktmerkmale (240) enthält und die Vielzahl von zweiten Kontaktmerkmalen (240) fünf Kontaktmerkmale enthält.

4. Anker nach einem der Ansprüche 1-3, wobei die erste (110) und die zweite Rahmenkomponente (120) durch mindestens eine Windung des einen oder der mehreren Drähte (350) verbunden sind.

5. Anker nach einem der Ansprüche 1-4, wobei die erste Rahmenkomponente (110) und die zweite Rahmenkomponente (120) derart nach innen zueinander gekrümmt sind, dass die Vielzahl von ersten äußeren Scheiteln (242) und die Vielzahl von zweiten äußeren Scheiteln (242) zueinander abgewinkelt sind, wenn sich der Anker (100) in einem uneingeschränkten Zustand befindet.

6. Anker nach einem der Ansprüche 1 bis 5, ferner umfassend eine Membran (460), die Flächen der ersten Rahmenkomponente (110) und der zweiten Rahmenkomponente (120) überspannt, die durch den einen oder die mehreren Drähte (350) definiert sind, die die erste Rahmenkomponente (110) und die zweite Rahmenkomponente (120) ausbilden.

## Revendications

1. Dispositif d'ancrage (100) pour un dispositif médical implantable, le dispositif d'ancrage (100) comprenant :
un ou plusieurs fils (350) agencés pour former :
un premier composant de cadre (110) comprenant une pluralité de premiers éléments de contact (240) définissant une pluralité de premiers sommets externes (242) et une pluralité de premiers sommets internes (244) agencés entre chacun de la pluralité de premiers éléments de contact (240) ; et
un second composant de cadre (120) comprenant une pluralité de seconds éléments de contact (240) définissant une pluralité de seconds sommets externes (242) et une pluralité de seconds sommets internes (244) agencés entre chacun de la pluralité de seconds éléments de contact (240),
dans lequel la pluralité de premiers éléments de contact (240) et la pluralité de seconds éléments de contact (240) sont positionnés l'un en face de l'autre de sorte que la pluralité de premiers éléments de contact (240) et la pluralité de seconds éléments de contact (240) soient alignés angulairement l'un par rapport à l'autre autour d'un axe longitudinal central du dispositif d'ancrage ;
le ou les fils comprenant une section sensiblement linéaire (246) s'étendant entre chacun de la pluralité de premiers sommets externes (242) et une section sensiblement linéaire s'étendant entre chacun de la pluralité de seconds sommets externes (242) de sorte que la pluralité de premiers éléments de contact (240) aient chacun approximativement une forme de losange et que la pluralité de seconds éléments de contact (240) aient chacun approximativement une forme de losange ; et
une section intermédiaire (130) comprenant une membrane couplant le premier composant de cadre (110) et le second composant de cadre (120), le premier composant de cadre (110) étant agencé sur un premier côté de la section intermédiaire (130) et le second composant de cadre (120) étant agencé sur un second côté de la section intermédiaire (130), les sommets internes (244) de chacun de la pluralité de premiers et seconds éléments de contact (240) étant adjacents à la section intermédiaire (130) et espacés autour d'une circonférence de celle-ci, dans lequel le ou les fils (350) comprennent un motif d'enroulement continu formant le premier composant de cadre (110), le second composant de cadre (120) et la section intermédiaire (130).

2. Dispositif d'ancrage de la revendication 1, dans lequel la pluralité de premiers éléments de contact (240) comprend un nombre impair d'éléments de contact et la pluralité de seconds éléments de contact (240) comprend un nombre impair d'éléments de contact.

3. Dispositif d'ancrage de la revendication 2, dans lequel la pluralité de premiers éléments de contact (240) comprend cinq éléments de contact (240) et la pluralité de seconds éléments de contact (240) comprend cinq éléments de contact.

4. Dispositif d'ancrage de l'une quelconque des revendications 1 à 3, dans lequel les premier (110) et second composants de cadre (120) sont reliés par au moins une spire du ou des fils (350).

5. Dispositif d'ancrage de l'une quelconque des revendications 1 à 4, dans lequel le premier composant de cadre (110) et le second composant de cadre (120) sont incurvés vers l'intérieur l'un vers l'autre de sorte que la pluralité de premiers sommets externes (242) et la pluralité de seconds sommets externes (242) soient inclinés l'un vers l'autre lorsque le dispositif d'ancrage (100) est dans un état non contraint.

6. Dispositif d'ancrage de l'une quelconque des revendications 1 à 5, comprenant en outre une membrane (460) recouvrant des zones du premier composant de cadre (110) et du second composant de cadre (120) définies par le ou les fils (350) qui forment le premier composant de cadre (110) et le second composant de cadre (120).
